# EUROPÄISCHE PATENTSCHRIFT

(11) **EP 1 303 199 B1**
(45) Veröffentlichungstag und Bekanntmachung des Hinweises auf die Patenterteilung: **16.11.2005**
(21) Anmeldenummer: 01942863.0
(22) Anmeldetag: 19.06.2001
(51) Int. Cl.: A23L 1/22, A61K 31/20, A23P 1/04

(54) **VERFAHREN ZUR HERSTELLUNG VON UNGESÄTTIGTEM FETTSÄURE-TROCKENKONZENTRAT**
METHOD FOR PRODUCING A DRY CONCENTRATE OF AN UNSATURATED FATTY ACID
PROCEDE POUR PRODUIRE UN CONCENTRE SEC D'ACIDES GRAS INSATURES

(30) Priorität: 20.06.2000 AT 10632000
(43) Veröffentlichungstag der Anmeldung: 23.04.2003
(73) Patentinhaber: vis-vitalis Lizenz- und Handels GmbH, 5020 Salzburg (AT)
(72) Erfinder: FUCHS, Norbert, A-5571 Mariapfarr (AT); KÖSSLER, Peter, A-5571 Mariapfarr (AT)
(74) Vertreter: Alge, Daniel
(86) Internationale Anmeldenummer: PCT/AT2001/000197
(87) Internationale Veröffentlichungsnummer: WO 2001/097634

(56) Entgegenhaltungen:
- EP-A- 0 607 886
- US-A- 6 030 645

## Beschreibung

Die Erfindung betrifft ein Verfahren zur Herstellung von ungesättigtem Fettsäure-Trockenkonzentrat sowie eine Zusammensetzung, umfassend zumindest eine ungesättigte Fettsäure und Lebensmittel, Getränke und Arzneimittel, umfassend die Zusammensetzung, vorgesehen.

Fettsäuren (Alkansäuren) sind Bausteine von Lipiden, Phosphoglyceriden, Glykolipiden, Cholesterinesterasen und Wachsen. Sie bestehen aus einer längeren, meist unverzweigten Kohlenwasserstoffkette und einer endständigen Carboxylgruppe. Die Kette ist entweder gesättigt oder enthält eine oder mehrere nichtkonjugierte cis-Doppelbindungen; letztere werden als ungesättigte Fettsäuren bezeichnet. Das für die Bildung ungesättigter Fettsäuren erforderliche Cytochrom-b₅-ADPH-abhängige Oxygenasesystem fehlt bei höheren Tieren, weshalb Linol- und Linolensäure für diese zu den essentiellen Fettsäuren gehören, d.h., dass deren Bedarf durch Nahrungsaufnahme gedeckt sein muss.

Während bis vor wenigen Jahren die Linolsäure (Omega-6-Fettsäure; C 18:2 (9,12)) in diätetischen Lebensmitteln als alleiniger wertbestimmender Faktor für die Zufuhr ungesättigter Fettsäuren galt, rückt nach neuen wissenschaftlichen Erkenntnissen die biologische und essentielle Bedeutung weiterer ungesättigter Fettsäuren in den Mittelpunkt, beispielsweise,Linolensäure, Stearidonsäure, Ölsäure, Erucasäure, Nervonsäure, Palmitoleinsäure und Vaccensäure. Diesen (hoch)ungesättigten Fettsäuren kommt eine hohe biologische und ernährungsmedizinische Bedeutung zu, insbesondere für den Prostaglandin- (also Entzündungs-)stoffwechsel, Herz- und Kreislauf, Kohlenhydratstoffwechsel (Diabetes), bei Übergewicht, für den Hautstoffwechsel (Neurodermitis, Psoriasis), Hormonstoffwechsel, für Leistungen des zentralen Nervensystems, Lungen (Asthma), Gelenke (Arthritis), Immunsystem (Allergien, Krebs, AIDS), Autoimmunerkrankungen, degenerative Erkrankungen der Gelenke, Wachstumsprozesse von Kindern und Jugendlichen, Stoffwechsel von Athleten und Schwerarbeitern und Alterungsprozessen. Für eine optimale Gesundheit ist demnach eine ausreichende und ausgewogene Zufuhr von (hoch)ungesättigten Fettsäuren notwendig.

Ungesättigte Fettsäuren kommen hauptsächlich in naturbelassenen Pflanzenölen in unterschiedlicher Konzentration vor, wobei diese durchwegs einen öligen Charakter aufweisen. Einige dieser Öle, insbesondere Hanföl, Leinöl und Fischöl, weisen zudem einen starken penetrierenden Eigengeruch und -geschmack auf, der eine breite Verwendung dieser Öle trotz ihres hohen gesundheitlichen Wertes weitgehend limitiert.

Ein weiteres Problem in Zusammenhang mit mit ungesättigten Fettsäuren angereicherten Lebensmitteln besteht darin, dass die naturbelassenen und somit die an ungesättigten Fettsäuren reichen Öle eine nur sehr eingeschränkte Haltbarkeit aufweisen und ihre ölige Konsistenz die Verwendbarkeit stark einschränkt. Um diese genannten Nachteile zu umgehen und mit ungesättigten Fettsäuren angereicherte Produkte, die auch von den Konsumenten akzeptiert werden, herzustellen, werden diese Öle technisch aufwendig verarbeitet. Durch die für diese Verarbeitung notwendigen strengen Produktionsbedingungen werden aber die ungesättigten Fettsäuren soweit verändert, dass sie ihren hohen gesundheitlichen Wert verlieren. Weiters befriedigt die Verarbeitung der genannten Öle in üblichen Arzneiformen, wie Kapseln, Tabletten oder Liquida, wiederum die ernährungsphysiologischen Tagesbedarfsmengen, die im Gramm-Bereich liegen, nur ungenügend, eben aufgrund der durch die angeführten Arzneiformen limitierten Dosis-Volumina.

In der WO 87/03899 ist ein Verfahren zur Herstellung eines Omega-3-Konzentrats beschrieben, wobei die Fettsäurefraktion von Fischöl bei Raumtemperatur verestert wird. Anschließend wird nach Erhitzung auf 55 bis 90°C und nachfolgender Kühlung auf 0°C der Alkylester präzipitiert und abgetrennt. Eine anschließende Reinigung durch Extraktion mit einem Lösungsmittel ergibt das gewünschte Produkt. Dieses Verfahren weist jedoch mehrere Schritte auf, in denen die ungesättigten Fettsäuren relativ strengen Bedingungen unterworfen werden (große Temperaturschwankungen, verschiedene Lösungsmittel und Puffer, etc.), sodass ein Teil der ungesättigten Fettsäuren verändert wird bzw. verlorengeht.

Die US 6 030 645 betrifft Trockenpartikel, umfassend eine oleophile, aktive Substanz, die in einem Trägermaterial dispergiert ist, wobei die Partikel mit einer Kalziumsilikat enthaltenden Zusammensetzung beschichtet sind. Die oleophile Substanz ist beispielsweise Arachidonsäure, Karotenoide etc. Als Trägermaterial wird u.a. Zellulose, Maltodextrin, Alginat, Lactat, Gummi, Gelatine, Zucker, Zuckeralkohol und Stärke angegeben. Diese Partikel werden durch ein Vermischen der oleophilen Substanz mit dem Trägermaterial hergestellt, wobei dieses Gemisch in das Kalziumsilikat gesprüht wird, so dass die durch das Sprühen entstandenen oleophilen Partikeln mit Kalziumsilikat beschichtet werden. Anschließend werden die Partikel getrocknet.

Gemäß der JP 6181725 A wird eine Zusammensetzung, umfassend eine leicht oxidierbare, ölige Substanz in einen porösen Träger durch Verringerung des Druckes so eingebracht, dass die Zusammensetzung die Luft in diesem porösen Träger verdrängt.

Die FR 2 758 055 A1 betrifft eine puderförmige Substanz, umfassend Öl auf der Basis von ungesättigten Fettsäuren und ein absorbierendes Agens, etwa Stärke. Das Öl und das absorbierende Agens werden homogenisiert und zerstäubt, um Mikropartikel zu erhalten, wonach das in diesen Mikropartikeln enthaltene Wasser evaporiert wird.

In der US 5 106 639 A ist ein Verfahren zur Herstellung von Nahrungsmittelzusätzen beschrieben, wobei ein Träger, ein Emulgator und ein Öl, umfassend Omega-3-Fettsäuren, vermischt und anschließend zu einem Puder getrocknet werden. Der Träger kann dabei z.B. Sojabohnenprotein, Stärke, Pektin, Gelatine, Kollagen, Kasein und ähnliches sein.

Die EP 0 424 578 A1 betrifft ein Trockengemisch, umfassend ein Öl mit ungesättigten Fettsäuren und Kaseinat, wobei diese zwei Substanzen miteinander vermischt werden, wonach die Zusammensetzung getrocknet wird.

In der DE 4 411 414 C1 wird ein Produkt zur enteralen Versorgung mit Fettsäuren und/oder Aminosäuren sowie ein Verfahren zur Herstellung dieses Produkts beschrieben, wobei die Fettsäuren durch eine gemeinsame Extrusion mit Stärke in die Amylosehelix eingelagert werden, so dass Einschlusskomplexe gebildet werden. Anschließend kann die Masse getrocknet werden.

Die US 4 559 222 A betrifft eine Zusammensetzung, umfassend ein Medikament, wobei diese Zusammensetzung weiters Mineralöl und Silikondioxid umfasst.

Die EP 0 607 886 A1 betrifft eine Parfumkomplex-Zusammensetzung umfassend u.a. ein Silikatmaterial zur reversiblen Adsorption des Parfumöls und mindestens eine ungesättigte Fettsäure. Dabei werden die Bestandteile miteinander vermischt und anschließend zum gewünschten Produkt ohne Trocknung weiterverarbeitet. Als Silikatmaterial ist u.a. eine amorphe Kieselsäure bzw. Kieselgel mit einer spezifischen Oberfläche von 100 bis 1000 m²/g beschrieben.

Diese oben beschriebenen Verfahren weisen jedoch den Nachteil auf, dass hochwertige Öle mit hohen Anteilen an ungesättigten Fettsäuren nicht ausreichend schonend behandelt werden, so dass ein zu hoher Anteil an ungesättigten Fettsäuren im Laufe des Verfahrens verlorengeht.

Aufgabe der vorliegenden Erfindung ist es, ein Verfahren zur Herstellung eines Konzentrats von ungesättigten Fettsäuren zur Verfügung zu stellen, in dem die oben genannten Nachteile umgangen werden, wobei jedoch der hohe gesundheitliche Wert der ungesättigten Fettsäuren beibehalten wird. Durch ein derartiges Konzentrat sollen Dosen mit einer ausreichenden Menge an ungesättigten Fettsäuren zur Verfügung gestellt werden, dass diese ein allzu großes Volumen aufweisen. Das Verfahren soll eine feine Oberflächenverteilung der Ölteilchen und eine exakte Dosierbarkeit der Mengenverhältnisse von Fettsäuren und Trägermatrix gewährleisten.

Das erfindungsgemäße Verfahren der eingangs angeführten Art ist dadurch gekennzeichnet, dass eine Substanz umfassend zumindest eine ungesättigte Fettsäure auf eine biologisch inerte Matrix mit großer Oberfläche von 50 bis 1000 m²/g aufgebracht und anschließend getrocknet wird. Unter "Substanz" wird im Rahmen der vorliegenden Erfindung Öl, vorzugsweise unbehandeltes Öl, sowie auch jegliche Zusammensetzung, umfassend zumindest eine ungesättigte Fettsäure, verstanden. Durch das Aufbringen der Substanz auf eine biologisch inerte Matrix mit großer Oberfläche wird erreicht, dass die Substanz auf möglichst geringem Volumen möglichst stark verteilt wird. Dadurch wird es möglich, die Substanz rasch und unter sanften Bedingungen zu trocknen und in hohen Konzentrationen lagerstabil zur Verfügung zu stellen. Dabei ist es wichtig, dass die Matrix biologisch inert ist und somit die ungesättigte(n) Fettsäure(n) nicht angegriffen oder verändert wird (werden). Die ungesättigten Fettsäuren haften auf der Matrix, wodurch sie 1) leicht handhabbar werden und 2) bietet die Matrix einen gewissen Schutz vor anderen, die ungesättigten Fettsäuren angreifenden Stoffen. Für eine gute Verteilung und ausreichende Trocknung der ungesättigten Fettsäuren ist es dabei wichtig, dass die biologisch inerte Matrix eine große Oberfläche aufweist, d.h. eine Oberfläche von 50-1000 m²/g.

Im Rahmen der vorliegenden Anmeldung ist unter einer Matrix mit "großer Oberfläche" ein Träger zu verstehen, der hochdispers ist. Durch das Aufbringen, etwa durch Aufsprühen der Fettsäuren auf die hochdisperse Matrix, werden feine Fettsäuretröpfchen auf die fein verteilten Matrix-Teilchen angelagert. Dadurch wird eine optimale feine Oberflächenverteilung der Fettsäureteilchen sowie eine exakte Dosierbarkeit der Mengenverhältnisse von Fettsäuren und Trägermatrix gewährleistet. Auf diese Weise ist es erstmals möglich, die Temperatur- und Sauerstoff-empfindlichen Fettsäuren schonend und ohne Verluste bzw. mit minimalen Verlusten zu konzentrieren und zu trocknen. Im Gegensatz zu den porösen Trägermaterialien gemäß dem Stand der Technik wird durch die hochdisperse Matrix ein trockenes, hochkonzentriertes Fettsäure-Produkt erhalten. Damit werden hochwertige Öle, etwa hochwertige Pflanzenöle mit hohen Anteilen an thermolabilen und sauerstoffempfindlichen Fettsäuren, temperaturschonend mit der Matrix vereinigt und getrocknet.

Die Matrix weist vorzugsweise eine durchschnittliche Oberfläche von mindestens 100 m²/g, besonders bevorzugt mindestens 150 m²/g, noch bevorzugter mindestens 200 m²/g, am meisten bevorzugt mindestens 400 m²/g, auf.

Die durchschnittliche Teilchengröße der Matrix beträgt beispielsweise etwa maximal 900 nm, vorzugsweise maximal 500 nm, besonders bevorzugt maximal 250 nm, maximal 100 nm, maximal 50 nm, maximal 25 nm und am meisten bevorzugt maximal 15 nm.

Die Kombination der ungesättigten Fettsäuren auf der biologisch inerten Matrix und anschließender Trocknung gewährleistet das zur Verfügung stellen eines Trockenkonzentrats mit ungesättigten Fettsäuren ohne ölige Konsistenz und penetrierendem Eigengeruch und -geschmack. Weiters ist dadurch eine wesentlich verbesserte Haltbarkeit gegeben. Zudem ist das erfindungsgemäße Herstellungsverfahren noch schnell und kostengünstig durchführbar ist. Schließlich lassen sich die erfindungsgemäßen Zusammensetzungen leicht und einfach lebensmitteltechnisch weiterverarbeiten.

Ein weiterer Vorteil besteht darin, dass es zu praktisch keinem Verlust der ungesättigten Fettsäuren wie bei den gängigen Verfahren, beispielsweise Extraktionsverfahren, kommt.

Dabei ist es besonders günstig, wenn die Substanz über Düsen auf die Matrix aufgebracht wird. Dadurch wird gewährleistet, dass bereits vor Aufbringen der Substanz auf die Matrix die Substanz fein verteilt wird und so eine gleichmäßige feine Verteilung auf der Matrix gewährleistet wird.

Für eine gründliche Mischung ist es günstig, wenn die Matrix-Fettsäuremischung in einer Mischanlage, insbesondere mittels Mischschnecke, vermischt wird. Dabei ist jede im Stand der Technik bekannte Mischanlage (mit Mischschnecke) einsetzbar, wobei der Mischbehälter völlig verschließbar sein sollte. Dabei ist es vorteilhaft, wenn zusätzlich an der Behälterwand Vibratoren angebracht sind, die die Einmischung von schwer zu mischenden Rohstoffen verbessert. Die Mischgenauigkeit kann weiters durch Kipp- und Schaukelbewegung des Mischbehälters erhöht werden. Durch Scherköpfe wird die Mischung verfeinert und Knollen und Agglomerate, die in der Mischmasse entstehen können, werden zerkleinert. Dabei ist es günstig, wenn Parameter wie Mischzeit, Einspritzzeit, Einspritzdruck, Kippwinkel, Vibratoren und Scherkopfzuschaltung programmierbar bzw. einstellbar sind. Dadurch ist die Verfahrensoptimierung für den Fachmann auf dem Gebiet der Lebensmitteltechnologie für alle Substanzen und Oberflächen leicht möglich. Ein Beispiel für eine geeignete Mischanlage ist der Batch-Mischer "Prodima AC-LI/500".

Für eine gleichmäßige sanfte und doch rasche Trocknung ist es vorteilhaft, wenn die Matrix-Fettsäuremischung vakuumgetrocknet wird. Dadurch können relativ sanfte Betriebsbedingungen eingehalten werden. Diese Vakuumtrocknung ist für den Fachmann allgemein bekannt. Während der Vakuumtrocknung kann die Mischung beispielsweise in einem Kessel mittels einem Rührwerk ständig gemischt werden. Der durch die Vakuumtrocknung entstehende Dampf kann mit einem Kondensator kondensiert und in einen Wasserbehälter abgeleitet werden. Der Kessel ist vorzugsweise drehbar und liegend vorgesehen und kann jegliche Größe aufweisen, beispielsweise 500 bis 1000 Liter. Vorzugsweise ist die Anlage temperatur- bzw. druckgesteuert.

Eine besonders günstige Trocknung ist dadurch gekennzeichnet, dass die Matrix-Fettsäuremischung bei 1-50 mbar, insbesondere bei 10-30 mbar, getrocknet wird. Bei diesem Vakuum ist eine schonende Trocknung ohne Temperaturschädigung gewährleistet.

Dabei ist es besonders vorteilhaft, wenn die Matrix-Fettsäuremischung bei 10 bis 50°C, insbesondere bei 30 bis 36°C, getrocknet wird. In diesem Arbeitsbereich kommt es zu keiner Schädigung der ungesättigten Fettsäuren. Der Kessel wird beispielsweise mittels Steuerung auf einer konstanten Temperatur beheizt. Dazu kann die Trommel mit einem Doppelmantel für Heizwasser versehen sein, welches über die Wärmerückgewinnung des Kühlaggregates beheizt wird. Weiters kann noch zusätzlich ein Durchlauferhitzer eingebaut sein, um zusätzlich Wärme zu erzeugen.

Vorzugsweise wird die Substanz auf eine Silicium-Matrix aufgebracht, beispielsweise eine SiO₂-Matrix. Diese Matrix ist biologisch absolut inert und weist weiters eine ausreichend große Oberfläche auf, um eine für das Verfahren günstige Matrix zur Verfügung zu stellen.

Dabei ist es besonders günstig, wenn die Substanz auf eine Silicium dioxydatum dispersum-Matrix aufgebracht wird. Diese Matrix eignet sich besonders gut für das Auftragen von ungesättigten Fettsäuren und anschließender Trocknung.

Beispielsweise ist die Matrix aus Aerosil® hergestellt, eine hochdisperse Kieselsäure von über 99,8 % SiO₂-Gehalt. Diese Matrix ist aus amorphen kugelförmigen Teilchen aufgebaut, die einen Durchmesser von etwa 10 bis 20 nm besitzen. Bei einem Volumen von ca. 15 ml besitzt 1 g Aerosil® eine Oberfläche von 100 bis 400 m². Diese Matrix eignet sich besonders gut für das erfindungsgemäße Verfahren.

Ein besonders vorteilhaftes Verfahrens ist dadurch gekennzeichnet, dass als Substanz Leinöl, Distelöl, Borretschöl, Hanföl, Sojaöl, Kürbiskernöl, Sonnenblumenöl, Sesamöl, Nachtkerzenöl und/oder Fischöl auf die Matrix aufgebracht wird. Diese Öle umfassen unterschiedliche Konzentrationen von (hoch)ungesättigten Fettsäuren (siehe Tabellen 1 bis 3). Werden diese Pflanzenöle in naturbelassenem Zustand auf die Matrix aufgebracht, weisen sie in Bezug auf ihren Gehalt an ungesättigten Fettsäuren einen ausgesprochen hohen gesundheitlichen Wert auf. Weiters wird dadurch das Verfahren rasch und kostengünstig durchgeführt.

Vorzugsweise wird eine Substanz umfassend (hoch)ungesättigte Fettsäuren, insbesondere Omega-3-, Omega-6-, Omega-7- und/oder Omega-9-Fettsäuren auf die Matrix aufgebracht. Es können entweder einzelne gereinigte Fettsäuren verwendet werden oder auch eine Mischung zweier oder mehrerer dieser Fettsäuren. Weiters kann auch ein Pflanzenöl umfassend diese Fettsäuren verwendet werden.

Ein besonders vorteilhaftes Verfahren ist dadurch gekennzeichnet, dass 1 bis 3, insbesondere 1,5, Gewichtsteile Substanz pro Gewichtsteil Matrix auf die Matrix aufgebracht wird. Dadurch wird ein ideales Verhältnis von ungesättigten Fettsäuren zur Matrix erhalten, wobei eine maximale Menge an Fettsäuren auf die dafür notwendige Menge Matrix aufgetragen wird, und eine möglichst große Oberfläche pro möglichst kleinem Volumen Fettsäure-Trockenkonzentrat erhalten wird.

Um ein maximal haltbares Produkt zu erhalten, ist es dabei günstig, wenn der Matrix-Fettsäuremischung weiters zumindest ein Stabilisator, insbesondere ein Antioxidans, zugesetzt wird. Insbesondere für hochungesättigte Fettsäuren eignen sich D,L-Alpha-tocopherol und Ascorbylpalmitat als Stabilisatoren. Dadurch wird die Haltbarkeit des Trockenkonzentrats erhöht und die Stabilität, insbesondere in Hinblick auf die weitere Verarbeitung, verbessert.

Besonders bevorzugt ist es weiters, wenn der Matrix-Fettsäuremischung zumindest ein Geruchs- und/oder Geschmackskorrigens zugesetzt wird. Auf diese Weise wird jeder möglicherweise verbleibende unangenehme Geruch oder Geschmack der ungesättigten Fettsäuren kupiert. Unter Geruchs- und Geschmackskorrigens wird im Rahmen der vorliegenden Erfindung nicht nur ein Kaschieren eines unangenehmen Geruchs oder Geschmacks verstanden, sondern auch das Zusetzen eines angenehmen Geruchs oder Geschmacks, beispielsweise ein Süßstoff, ein Fruchtgeschmack, ätherische Öle, etc.

Dabei ist es besonders günstig, wenn als Geruchs- oder Geschmackskorrigens Etheroleum citri zugesetzt wird. Dieser eignet sich besonders gut für den Zusatz zu ungesättigten Fettsäuren.

Ein weiteres vorteilhaftes Verfahren ist dadurch gekennzeichnet, dass der Matrix-Fettsäuremischung vor der Trocknung Milch, insbesondere pasteurisierte Milch, zugesetzt wird. Es kann jegliche Milch zugesetzt werden, insbesondere Kuh-, Stuten-, Esels-, Colostral-, Ziegen- und/oder Schafsmilch. Trockenkonzentrate aus den genannten Milch-Spezies oder Fraktionen aus diesen weisen u.a. immunstimulierende Effekte auf den humanen und tierischen Organismus auf. Durch den Zusatz von (hoch)ungesättigten Fettsäuren kann der Gehalt an diesen essentiellen Nährstoffen in Milchkonzentraten in bedarfsadäquaten Mengen erhöht und damit der biologische Wert von Milchkonzentraten verbessert werden.

Vorzugsweise werden 1 bis 2, insbesondere ca. 1,5, Gewichtsteile Milch pro Gewichtsteil Matrix-Fettsäuremischung zugesetzt. Dieses Mengenverhältnis hat sich als besonders günstig erwiesen, wobei die Vorteile des Zusatzes der Milch erhalten werden, ohne dabei den weiteren Trocknungsprozeß zu stören bzw. negative Auswirkungen auf die ungesättigten Fettsäuren zu erhalten.

Für die Einnahme dieser Konzentrate ist es besonders günstig, wenn die Matrix-Fettsäuremischung nach der Trocknung weiterverarbeitet wird, insbesondere zu Pulver, Kapseln, Tabletten oder Liquida. Dabei können auch weitere Zusatzstoffe hinzugefügt werden, etwa Vitamine, Geschmacksstoffe, Mineralstoffe, Arzneimittel, etc.. Weiters kann beispielsweise die notwendige Tagesdosis an ungesättigten Fettsäuren zu einer Einheit, wie Tablette oder Kapsel, verarbeitet werden.

Dabei ist es besonders bevorzugt, wenn die Matrix-Fettsäuremischung nach der Trocknung zu Lebensmitteln und/oder Getränken zugesetzt wird, insbesondere zu Babynahrung, Milchprodukten und/oder Backmischungen. Da das Fettsäure-Trockenkonzentrat keine ölige Konsistenz und keinen unangenehmen Geruch oder Geschmack aufweist, wird durch dessen Zusatz das jeweilige Lebensmittel oder Getränk nicht wesentlich verändert, sodass keine oder kaum zusätzliche Verfahrensschritte notwendig sind.

Ein weiterer Aspekt der vorliegenden Erfindung betrifft eine Zusammensetzung, die nach dem oben beschriebenen erfindungsgemäßen Verfahren erhältlich ist, umfassend zumindest eine ungesättigte Fettsäure und eine biologisch inerte Matrix mit großer Oberfläche von 50 bis 1000 m²/g. Hinsichtlich der ungesättigten Fettsäure und der Matrix gilt wiederum das oben bereits Genannte, wobei die Zusammensetzung vorzugsweise ein Konzentrat von zumindest einer ungesättigten Fettsäure ist. Diese Zusammensetzung kann jede erdenkliche Konsistenz aufweisen, vorzugsweise jedoch wird sie als Trockenkonzentrat zur Verfügung gestellt. Die Zusammensetzung weist durch die Kombination der ungesättigten Fettsäure und der Matrix mit großer Oberfläche überraschenderweise keine ölige Konsistenz auf, die bei den gängigen Produkten umfassend ungesättigte Fettsäuren in der Regel üblich ist. Weiters weist diese Zusammensetzung auch nicht den sonst vorkommenden penetrierenden Eigengeruch und Geschmack auf, und ist, insbesondere als Trockenkonzentrat, ausgesprochen lange haltbar.

Vorzugsweise ist die Matrix eine Silicium-Matrix, beispielsweise eine SiO₂-Matrix, insbesondere eine Silicium dioxydatum dispersum-Matrix. Diese Matrix eignet sich besonders gut als inerte Grundsubstanz einer essentielle Fettsäuren umfassenden Zusammensetzung, weist eine große Oberfläche auf und eignet sich ausgesprochen gut für jede weitere Verarbeitung.

Vorzugsweise umfasst die Zusammensetzung Leinöl, Distelöl, Borretschöl, Hanföl, Sojaöl, Kürbiskernöl, Sonnenblumenöl, Sesamöl, Nachtkerzenöl und/oder Fischöl. Diese Öle weisen einen hohen Gehalt an (hoch)-ungesättigten Fettsäuren und insbesondere in naturbelassenem zustand einen hohen gesundheitlichen Wert auf.

Dabei ist es besonders günstig, wenn die Zusammensetzung zumindest eine hochungesättigte Fettsäure, insbesondere Omega-3-, Omega-6-, Omega-7- und/oder Omega-9-Fettsäuren, umfasst. Diese essentiellen Fettsäuren sind besonders wichtig für zahlreiche biochemische Prozesse und auch für den Aufbau von lebenswichtigen Stoffen.

Vorteilhafterweise umfasst die Zusammensetzung 0,1 bis 3 Gewichtsteile der zumindest einen ungesättigten Fettsäure pro Gewichtsteil Matrix. Selbstverständlich kann, falls naturbelassenes Öl für die Herstellung der Zusammensetzung verwendet wird, die Konzentration und auch die Art der ungesättigten Fettsäuren mehr oder weniger stark variieren. Dieses Verhältnis, das natürlich auch von der jeweiligen Weiterverarbeitung abhängt, ist jedoch in der Regel optimal.

Vorzugsweise umfasst die Zusammensetzung zumindest einen Stabilisator, insbesondere ein Antioxidans, zumindest ein Geruchs- und/oder Geschmackskorrigens, insbesondere Etheroleum citri, und/ oder getrocknete Milch, insbesondere pasteurisierte Milch. Diese Zusatzstoffe optimieren die Eigenschaften der Zusammensetzung, vereinfachen deren Weiterverarbeitung und wirken sich besonders günstig auf das Endprodukt, beispielsweise hinsichtlich Haltbarkeit bzw. Geschmack, aus.

Eine besonders günstige Zusammensetzung ist dadurch gekennzeichnet, dass sie 1 bis 2, insbesondere ca. 1,5, Gewichtsteile Milch pro Gewichtsteil Matrix-Fettsäuremischung umfasst. Dadurch wird eine optimale Konsistenz erreicht.

Für eine gute Haltbarkeit und einfache Weiterverarbeitung ist es bevorzugt, dass die Zusammensetzung getrocknet ist. Besonders bevorzugt sind dabei erfindungsgemäße Zusammensetzungen mit einem A_{w}-Wert von unter 0,8.

Ein weiterer Aspekt der vorliegenden Erfindung ist es, ein Lebensmittel, ein Getränk oder ein Arzneimittel zur Verfügung zu stellen, das dadurch gekennzeichnet ist, dass es mit einer erfindungsgemäßen Zusammensetzung, wie oben beschrieben, versetzt ist. Das Lebensmittel oder Getränk kann jegliches im Handel erhältliche Produkt sein, beispielsweise ein Grundnahrungsmittel oder ein Genußmittel. Die Konsistenz des Lebensmittels ist dabei nicht wesentlich, es kann sowohl flüssig, wie z.B. ein Fruchtsaft, zähflüssig, wie Jogurt, Marmelade, Öl, etc., oder fest, wie eine Backmischung, Müsli oder Ähnliches, sein. Selbstverständlich kann die Zusammensetzung auch in hochkonzentrierter Form als Brausetablette, Sirup oder Ähnliches zur Verfügung gestellt werden. Auch das Arzneimittel, das die erfindungsgemäße Zusammensetzung umfasst, kann jede erdenkliche Form und Konsistenz aufweisen, beispielsweise eine Form als Tablette, als Liquida, Pulver oder Kapsel.

Das erfindungsgemäße Verfahren wird nun anhand des nachfolgenden Beispiels, auf das es jedoch nicht beschränkt ist, näher erläutert.

### Beispiel :

In einem Mischer der Marke Prodima "Batch-Mischer Prodima AC-LI/500", in dem Silicium dioxydatum dispersum vorgegeben ist, werden unter ständigem Rühren die folgenden Komponenten über feine Düsen in die Spritzanlage eingespritzt:
- Leinöl: 2 Gewichtsteile (siehe Tabelle 1)
- Distelöl: 1 Gewichtsteil (siehe Tabelle 2)
- Borretschöl: 1 Gewichtsteil (siehe Tabelle 3)
- D,L-Alphatocopherol und Ascorbylpalmitat als Stabilisatoren
- Geruchs- und Geschmackskorrigenzien (Etheroleum citri)

**Tabelle 1:**

| Leinöl (g Fettsäuren/100 g Fett) | |
|---|---|
| Palmitinsäure | 5,95 |
| Stearinsäure | 3,60 |
| Ölsäure | 18,20 |
| Linolsäure | 13,90 |
| Linolensäure | 54,20 |

**Tabelle 2:**

| Distelöl - Fettsäurezusammensetzung % (unterliegt Schwankungen) | |
|---|---|
| C14:0 | 0,1 - 0,2 |
| C14:1 | 0 |
| C16:0 | 6,7 - 7,7 |
| C16:1 | 0 |
| C18:0 | 2,4 - 2,7 |
| C18:1 | 12,6 - 13,6 |
| C18:2 | 75,7 - 77,1 |
| C18:3 | 0 - 0,2 |
| C20:0 | 0,3 - 0,4 |
| C20:1 | 0 - 0,2 |
| C22:0 | 0 - 0,2 |
| C22:1 | 0 |
| C24:0 | 0 |

**Tabelle 3:**

| Borretschöl | |
|---|---|
| Säurewert (mg KOH/g Öl) | 0,1 % |
| gamma-Linolensäuregehalt | 23,6 % |

Das in der Mischanlage vorgegebene Siliciumdioxid weist im Verhältnis dazu 2,7 Gewichtsteile auf. Das so erhaltene EFS-Pulver (EFS = Essentielle Fettsäuren) weist einen Gesamtanteil von 48,3 % an essentiellen Fettsäuren mit folgendem Verteilungsmuster auf:

**Tabelle 4:**

| **W-3 Fettsäuren:** | |
|---|---|
| C 18:3 (9, 12, 15) Alpha-Linolensäure | 10,5 % |
| C 18:4 (4, 8, 12, 15) Stearidonsäure | 0,01 % |
| | |

| **W-6 Fettsäuren:** | |
|---|---|
| C 18:2 (9, 12) Linolsäure | 16,8 % |
| C 18:3 (6, 9, 12) Gamma-Linolensäure | 2,5 % |
| | |

| **W-9 Fettsäuren:** | |
|---|---|
| C 18:1 (9) Ölsäure | 7,8 % |
| C 22:1 (13) Erucasäure | 0,3 % |
| C 24:1 (15) Nervonsäure | 0,12 % |
| | |

| **W-7 Fettsäuren:** | |
|---|---|
| C 16:1 (9) Palmitoleinsäure | 0,02 % |
| C 18:1 (11) Vaccensäure | 0,05 % |

In einer Evaporations-Apparatur "Stutenmilchanlage" wurden ca. 41 kg EFS-Konzentrat vorgegeben. Anschließend wurden ca. 57 kg pasteurisierte Milch (wahlweise Kuh-, Stuten-, Esels-, Colostral-, Ziegen- oder Schafsmilch) beigegeben und 24 Stunden lang unter einer Temperatur von 32°C unter Vakuumbedingungen (ca. 10 mbar) evaporiert. Nach Ablauf von 24 Stunden war der wässrige Anteil der zugegebenen Milch temperaturschonend abgedampft. Der durch dieses Verfahren gewonnene Rückstand war ein Milch-EFS-Konzentrat mit hohem Anteil an (hoch)ungesättigten Fettsäuren in stabiler, organoleptisch akzeptabler und hochkonzentrierter Pulverform. Diese Pulverform kann zu verschiedenen Produkten (Backmischungen; Babynahrung, Milchprodukte) und auch in verschiedene Arzneiformen, wie Kapseln, Tabletten, etc., weiterverarbeitet werden.

## Patentansprüche

1. verfahren zur Herstellung von ungesättigtem Fettsäure-Trockenkonzentrat, **dadurch gekennzeichnet, dass** eine Substanz, umfassend zumindest eine ungesättigte Fettsäure, auf eine biologisch inerte Matrix mit großer Oberfläche von 50 bis 1000 m²/g aufgebracht und anschließend getrocknet wird.

2. Verfahren nach Anspruch 1, **dadurch gekennzeichnet, dass** die Substanz über Düsen auf die Matrix aufgebracht wird.

3. Verfahren nach Anspruch 1 oder 2, **dadurch gekennzeichnet, dass** die Matrix-Fettsäuremischung in einer Mischanlage, insbesondere mittels Mischschnecke, vermischt wird.

4. Verfahren nach Anspruch 3, **dadurch gekennzeichnet, dass** die Matrix-Fettsäuremischung vakuumgetrocknet wird.

5. Verfahren nach Anspruch 4, **dadurch gekennzeichnet, dass** die Matrix-Fettsäuremischung bei 1-50 mbar, insbesondere bei 10-30 mbar, getrocknet wird.

6. Verfahren nach Anspruch 4 oder 5, **dadurch gekennzeichnet, dass** die Matrix-Fettsäuremischung bei 10 bis 50°C, insbesondere bei 30 bis 36°C, getrocknet wird.

7. Verfahren nach einem der Ansprüche 1 bis 6, **dadurch gekennzeichnet, dass** die Substanz auf eine Silicium-Matrix aufgebracht wird.

8. Verfahren nach Anspruch 7, **dadurch gekennzeichnet, dass** die Substanz auf eine Silicium dioxydatum dispersum-Matrix aufgebracht wird.

9. Verfahren nach einem der Ansprüche 1 bis 8, **dadurch gekennzeichnet, dass** als Substanz Leinöl, Distelöl, Borretschöl, Hanföl, Sojaöl, Kürbiskernöl, Sonnenblumenöl, Sesamöl, Nachtkerzenöl und/oder Fischöl auf die Matrix aufgebracht wird.

10. Verfahren nach einem der Ansprüche 1 bis 9, **dadurch gekennzeichnet, dass** eine Substanz umfassend hochungesättigte Fettsäuren, insbesondere Omega-3-, Omega-6-, Omega-7- und/oder Omega-9-Fettsäuren, auf die Matrix aufgebracht wird.

11. Verfahren nach einem der Ansprüche 1 bis 10, **dadurch gekennzeichnet, dass** 1 bis 3, insbesondere 1,5, Gewichtsteile Substanz pro Gewichtsteil Matrix auf die Matrix aufgebracht werden.

12. Verfahren nach einem der Ansprüche 1 bis 11, **dadurch gekennzeichnet, dass** der Matrix-Fettsäuremischung weiters zumindest ein Stabilisator, insbesondere ein Antioxidans, zugesetzt wird.

13. Verfahren nach einem der Ansprüche 1 bis 12, **dadurch gekennzeichnet, dass** der Matrix-Fettsäuremischung weiters zumindest ein Geruchs- und/oder Geschmackskorrigens zugesetzt wird.

14. Verfahren nach Anspruch 13, **dadurch gekennzeichnet, dass** der Matrix-Fettsäuremischung als Geruchs- und Geschmackskorrigens Etheroleum citri zugesetzt wird.

15. Verfahren nach einem der Ansprüche 1 bis 14, **dadurch gekennzeichnet, dass** der Matrix-Fettsäuremischung vor der Trocknung Milch, insbesondere pasteurisierte Milch, zugesetzt wird.

16. Verfahren nach Anspruch 15, **dadurch gekennzeichnet, dass** 1 bis 2, insbesondere ca. 1,5, Gewichtsteile Milch pro Gewichtsteil Matrix-Fettsäuremischung zugesetzt werden.

17. Verfahren nach einem der Ansprüche 1 bis 16, **dadurch gekennzeichnet, dass** die Matrix-Fettsäuremischung nach der Trocknung weiterverarbeitet wird, insbesondere zu Pulver, Kapseln, Tabletten oder Liquida.

18. Verfahren nach einem der Ansprüche 1 bis 17, **dadurch gekennzeichnet, dass** die Matrix-Fettsäuremischung nach der Trocknung zu Lebensmitteln und/oder Getränken zugesetzt wird, insbesondere zu Babynahrung, Milchprodukten und/oder Backmischungen.

19. Zusammensetzung, **dadurch gekennzeichnet, dass** sie nach einem Verfahren gemäß einem der Ansprüche 1 bis 18 erhältlich ist, und dass sie zumindest eine ungesättigte Fettsäure und eine biologisch inerte Matrix mit großer Oberfläche von 50 bis 1000 m²/g umfasst.

20. Zusammensetzung nach Anspruch 19, **dadurch gekennzeichnet, dass** die Matrix eine Silicium-Matrix ist.

21. Zusammensetzung nach Anspruch 20, **dadurch gekennzeichnet, dass** die Matrix eine Silicium dioxydatum dispersum-Matrix ist.

22. Zusammensetzung nach einem der Ansprüche 19 bis 21, **dadurch gekennzeichnet, dass** sie Leinöl, Distelöl, Borretschöl, Hanföl, Sojaöl, Kürbiskernöl, Sonnenblumenöl, Sesamöl, Nachtkerzenöl und/oder Fischöl umfasst.

23. Zusammensetzung nach einem der Ansprüche 19 bis 22, **dadurch gekennzeichnet, dass** sie zumindest eine hochungesättigte Fettsäure, insbesondere Omega-3-, Omega-6-, Omega-7- und/oder Omega-9-Fettsäuren, umfasst.

24. Zusammensetzung nach einem der Ansprüche 19 bis 23, **dadurch gekennzeichnet, dass** sie 0,1 bis 3 Gewichtsteile der zumindest einen ungesättigten Fettsäure pro Gewichtsteil Matrix umfasst.

25. Zusammensetzung nach einem der Ansprüche 19 bis 24, **dadurch gekennzeichnet, dass** sie zumindest einen Stabilisator, insbesondere ein Antioxidans, umfasst.

26. Zusammensetzung nach einem der Ansprüche 19 bis 25, **dadurch gekennzeichnet, dass** sie zumindest ein Geruchs- und/oder Geschmackskorrigens umfasst.

27. Zusammensetzung nach Anspruch 26, **dadurch gekennzeichnet, dass** sie als Geruchs- und Geschmackskorrigens Etheroleum citri umfasst.

28. Zusammensetzung nach einem der Ansprüche 19 bis 27, **dadurch gekennzeichnet, dass** sie getrocknete Milch, insbesondere pasteurisierte Milch, umfasst.

29. Zusammensetzung nach Anspruch 28, **dadurch gekennzeichnet, dass** sie 1 bis 2, insbesondere ca. 1,5, Gewichtsteile Milch pro Gewichtsteil Matrix-Fettsäuremischung umfasst.

30. Zusammensetzung nach einem der Ansprüche 19 bis 29, **dadurch gekennzeichnet, dass** sie getrocknet ist.

31. Lebensmittel, **dadurch gekennzeichnet, dass** es mit einer Zusammensetzung nach einem der Ansprüche 19 bis 30 versetzt ist.

32. Getränk, **dadurch gekennzeichnet, dass** es mit einer Zusammensetzung nach einem der Ansprüche 19 bis 30 versetzt ist.

33. Arzneimittel, **dadurch gekennzeichnet, dass** es mit einer Zusammensetzung nach einem der Ansprüche 19 bis 30 versetzt ist.

## Claims

1. A method of preparing an unsaturated fatty acid dry concentrate, **characterised in that** a substance comprising at least one unsaturated fatty acid is applied on a biologically inert matrix having a large surface area of from 50 to 1000 m²/g, and is subsequently dried.

2. A method according to claim 1, **characterised in that** the substance is applied on the matrix through jet nozzles.

3. A method according to claim 1 or 2, **characterised in that** the matrix-fatty acid mixture is blended in a mixing device, in particular by the aid of a mixing screw.

4. A method according to claim 3, **characterised in that** the matrix-fatty acid mixture is vacuum-dried.

5. A method according to claim 4, **characterised in that** the matrix-fatty acid mixture is dried at 1-50 mbars, in particular 10-30 mbars.

6. A method according to claim 4 or 5, **characterised in that** the matrix-fatty acid mixture is dried at 10 to 50°C, in particular 30 to 36°C.

7. A method according to any one of claims 1 to 6, **characterised in that** the substance is applied on a silicon matrix.

8. A method according to claim 7, **characterised in that** the substance is applied on a *Silicium dioxydatum* dispersum-matrix.

9. A method according to any one of claims 1 to 8, **characterised in that** linseed oil, safflower oil, borage oil, hempseed oil, soybean oil, pumpkin seed oil, sunflower oil, sesame seed oil, evening primrose oil and/or fish oil are applied on the matrix as said substance.

10. A method according to any one of claims 1 to 9, **characterised in that** a substance comprising highly unsaturated fatty acids, in particular omega-3, omega-6, omega-7 and/or omega-9 fatty acids is applied on the matrix.

11. A method according to any one of claims 1 to 10, **characterised in that** 1 to 3, in particular 1.5, parts by weight of the substance per part by weight of the matrix are applied on the matrix.

12. A method according to any one of claims 1 to 11, **characterised in that** at least one stabilizer, in particular an antioxidant, is further added to the matrix-fatty acid mixture.

13. A method according to any one of claims 1 to 12, **characterised in that** at least one odour and/or taste corrective is further added to the matrix - fatty acid mixture.

14. A method according to claim 13, **characterised in that** *Etheroleum citri* is added to the matrix-fatty acid mixture as said odour and/or taste corrective.

15. A method according to any one of claims 1 to 14, **characterised in that** milk, in particular pasteurised milk, is added to the matrix-fatty acid mixture prior to drying.

16. A method according to claim 15, **characterised in that** 1 to 2, in particular about 1.5, parts by weight of milk are added per part by weight of the matrix-fatty acid mixture.

17. A method according to any one of claims 1 to 16, **character** **ised in that** the matrix-fatty acid mixture after drying is further processed, in particular, to form powders, capsules, tablets or liquids.

18. A method according to any one of claims 1 to 17, **characterised in that** the matrix-fatty acid mixture after drying is added to foods and/or beverages, in particular baby food, milk products and/or baking mixtures.

19. A composition, **characterised in that** it is obtainable according to a method according to any one of claims 1 to 18, and that it comprises at least one unsaturated fatty acid and a biologically inert matrix having a large surface area of from 50 to 1000 m²/g.

20. A composition according to claim 19, **characterised in that** the matrix is a silicon matrix.

21. A composition according to claim 20, **characterised in that** the matrix is a *Silicium dioxydatum* dispersum-matrix.

22. A composition according to any one of claims 19 to 21, **characterised in that** it comprises linseed oil, safflower oil, borage oil, hempseed oil, soybean oil, pumpkin seed oil, sunflower oil, sesame seed oil, evening primrose oil and/or fish oil.

23. A composition according to any one of claims 19 to 22, **characterised in that** it comprises at least one highly unsaturated fatty acid, in particular omega-3, omega-6, omega-7 and/or omega-9 fatty acids.

24. A composition according to any one of claims 19 to 23, **characterised in that** it comprises 0.1 to 3 parts by weight of said at least one unsaturated fatty acid per part by weight of the matrix.

25. A composition according to any one of claims 19 to 24, **characterised in that** it comprises at least one stabilizer, in particular an antioxidant.

26. A composition according to any one of claims 19 to 25, **characterised in that** it comprises at least one odour and/or taste corrective.

27. A composition according to claim 26, **characterised in that** it comprises Etheroleum citri as said odour and/or taste corrective.

28. A composition according to any one of claims 19 to 27, **characterised in that** it comprises dried milk, in particular pasteurised milk.

29. A composition according to claim 28, **characterised in that** it comprises 1 to 2, in particular about 1.5, parts by weight of milk per part by weight of the matrix-fatty acid mixture.

30. A composition according to any one of claims 19 to 29, **characterised in that** it is dried.

31. A food, **characterised in that** it is supplemented with a composition according to any one of claims 19 to 30.

32. A beverage, **characterised in that** it is supplemented with a composition according to any one of claims 19 to 30.

33. A drug, **characterised in that** it is supplemented with a composition according to any one of claims 19 to 30.

## Revendications

1. Procédé pour la fabrication de concentré sec d'acide gras insaturé, **caractérisé en ce qu'**une substance, comprenant au moins un acide gras insaturé, est appliquée sur une matrice biologiquement inerte d'une surface importante de 50 à 1000 m²/g puis séchée.

2. Procédé selon la revendication 1, **caractérisé en ce que** la substance est appliquée sur la matrice par l'intermédiaire d'une buse.

3. Procédé selon la revendication 1 ou la revendication 2, **caractérisé en ce que** le mélange acide gras-matrice est mélangé dans un dispositif mélangeur, en particulier au moyen d'une mélangeuse à vis.

4. Procédé selon la revendication 3, **caractérisé en ce que** le mélange acide gras-matrice est séché sous vide.

5. Procédé selon la revendication 4, **caractérisé en ce que** le mélange acide gras-matrice est séché à 1-50 mbar, en particulier à 10-30 mbar.

6. Procédé selon la revendication 4 ou 5, **caractérisé en ce que** le mélange acide gras-matrice est séché de 10 à 50°C, en particulier entre de 30 à 36°C.

7. Procédé selon l'une des revendications 1 à 6, **caractérisé en ce que** la substance est appliquée sur une matrice de silicium.

8. Procédé selon la revendication 7, **caractérisé en ce que** la substance est appliquée sur une matrice de dioxyde de silicium dispersé.

9. Procédé selon l'une des revendications 1 à 8, **caractérisé en ce que** la substance appliquée sur la matrice est de l'huile de lin, de l'huile de chardon, de l'huile de bourrache, de l'huile de chanvre, de l'huile de soja, de l'huile de pépin de courge, de l'huile de tournesol, de l'huile de sésame, de l'huile d'onagre et/ou de l'huile de poisson.

10. Procédé selon l'une des revendications 1 à 9, **caractérisé en ce qu'**une substance comprenant des acides gras poly insaturés, en particulier des acides gras oméga-3, oméga-6, oméga-7 et/ou oméga-9, est appliquée sur la matrice.

11. Procédé selon l'une des revendications 1 à 10, **caractérisé en ce que** 1 à 3, en particulier 1,5 unité de poids de substance est appliquée sur la matrice par unité de poids de matrice.

12. Procédé selon l'une des revendications 1 à 11, **caractérisé en ce qu'**un stabilisateur au moins, en particulier un antioxydant, est ajouté en outre au mélange acide gras-matrice.

13. Procédé selon l'une des revendications 1 à 12, **caractérisé en ce qu'**un correcteur de goût et/ou d'odeur est au moins ajouté au mélange acide gras-matrice.

14. Procédé selon la revendication 13, **caractérisé en ce que** de l'huile essentielle de citron est ajoutée en outre comme correcteur de goût ou d'odeur au mélange acide gras-matrice.

15. Procédé selon l'une des revendications 1 à 14, **caractérisé en ce que** du lait, en particulier du lait pasteurisé, est ajouté au mélange acide gras-matrice avant le séchage.

16. Procédé selon la revendication 15, **caractérisé en ce que** 1 à 2, en particulier environ 1,5 unités de poids de lait est ajoutée par unité de poids du mélange acide gras-matrice.

17. Procédé selon l'une des revendications 1 à 16, **caractérisé en ce que** le mélange acide gras-matrice est encore transformé après le séchage, en particulier en poudre, en capsule, en comprimé ou en liquide.

18. Procédé selon l'une des revendications 1 à 17, **caractérisé en ce que** le mélange acide gras-matrice est ajouté après le séchage à des aliments et/ou des boissons, en particulier pour l'alimentation des bébés, les produits laitiers et/ou des préparations pour produits de boulangerie.

19. Composition, **caractérisée en ce qu'**elle peut être obtenue par un procédé selon l'une des revendications 1 à 18, et **en ce qu'**elle comprend au moins un acide gras insaturé et une matrice biologiquement inerte d'une surface importante allant de 50 à 1000 m²/g.

20. Composition selon la revendication 19, **caractérisée en ce que** la matrice est une matrice en silicium.

21. Composition selon la revendication 20, **caractérisée en ce que** la matrice est une matrice en dioxyde de silicium dispersé.

22. Composition selon l'une des revendications 19 à 21, **caractérisée en ce qu'**elle comprend de l'huile de lin, de l'huile de chardon, de l'huile de bourrache, de l'huile de chanvre, de l'huile de soja, de l'huile de pépin de courge, de l'huile de tournesol, de l'huile de sésame, de l'huile d'onagre et/ou de l'huile de poisson.

23. Composition selon l'une des revendications 19 à 22, **caractérisée en ce qu'**elle comprend au moins un acide gras hautement poly insaturé, en particulier les acides gras oméga 3, oméga 6, oméga 7 et/ou oméga 9.

24. Composition selon l'une des revendications 19 à 23, **caractérisée en ce qu'**elle comprend 0,1 à 3 unités de poids d'au moins un acide gras insaturé par unité de poids de matrice.

25. Composition selon l'une des revendications 19 à 24, **caractérisée en ce qu'**elle comprend au moins un stabilisateur, en particulier un anti-oxydant.

26. Composition selon l'une des revendications 19 à 25, **caractérisée en ce qu'**elle comprend au moins un correcteur de goût et/ou d'odeur.

27. Composition selon la revendication 26, **caractérisée en ce qu'**elle comprend de l'huile essentielle de citron comme correcteur de goût et d'odeur.

28. Composition selon l'une des revendications 19 à 27, **caractérisée en ce qu'**elle comprend du lait lyophilisé, en particulier du lait pasteurisé.

29. Composition selon la revendication 28, **caractérisée en ce qu'**elle comprend de 1 à 2, en particulier environ 1,5 unité de poids de lait par unité de poids de mélange acide gras-matrice.

30. Composition selon l'une des revendications 19 à 29, **caractérisée en ce qu'**elle est séchée.

31. Aliment, **caractérisé en ce qu'**il est mélangé avec une composition selon l'une des revendications 19 à 30.

32. Boisson, **caractérisée en ce qu'**elle est mélangée avec une composition selon l'une des revendications 19 à 30.

33. Médicament, **caractérisé en ce qu'**il est mélangé avec une composition selon l'une des revendications 19 à 30.
